# EUROPEAN PATENT APPLICATION

(11) **EP 1 717 575 A1**
(43) Date of publication of application: **02.11.2006**
(21) Application number: 05076561.9
(22) Date of filing: 06.07.2005
(51) Int. Cl.: G01N 27/04, G01N 33/38

(54) **Method for internal testing of materials**

(30) Priority: 27.04.2005 NL 1028886
(71) Applicant: Nederlandse Organisatie voor Toegepast-Natuurwetenschappelijk Onderzoek TNO, 2628 VK Delft (NL)
(72) Inventor: Peelen, Wilhelmus Hendrikus Anna, 3117 XC Schiedam (NL)
(74) Representative: Winckels, Johannes Hubertus F.

(57) **Abstract**

Method to examine the internal condition, e.g. moisture, of a material (1). From electrodes (2), applied to the surface of the material to be examined, the mutual electrical conductivity is measured. The measured values are input to a model (6) in which the measured electrical values are converted into a moisture distribution in the interior of the material, e.g. by using, departing from a theoretical connection between both, a 'best fit' algorithm.

## Description

### Field

The invention concerns a method for examining the internal condition of a material, e.g. the moisture distribution in architectural constructions, works of art etc.

### Background

A known method with which moisture distribution in the depth direction of porous building materials can be determined, is based on a sensor, called Multi Ring Electrode (MRE), which is applied in existing constructions via a drill hole and which can be poured in at new concrete constructions. The sensor which is applied in the object to be monitored in that way, comprises a series of ring-shaped electrodes which are separated by insulators, the electrical resistance (or impedance) being measured between the successive electrodes. These resistance values can be converted to a curve for the course of the moisture content in the material with the help of calibration curves. Because the electrodes are situated at different depths in the material, a moisture distribution in depth direction can be measured.

The known method has several disadvantages. In the first place, to be able to measure at existing constructions, a hole has to be drilled in the material in which the sensor is placed. What's more, good contact between the sensor and the material has to be provided. For that reason an adhesive layer has to be used which, however, influences the measurement. Correction for this influence is difficult and not always reliable. Secondly, the known method is a destructive measuring method, which is not allowed for e.g. monuments etc. In the third place, it is not possible to do predictions of the moisture distribution with the known sensor system.

### Summary

The present invention aims to meet the objections of the known method resp. system. To that end the invention provides a non-destructive measuring method for the measurement and prediction of e.g. the moisture distribution in building materials.

So hereinafter the new method is presented and discussed more in detail below, for examining (measuring, monitoring) the internal condition (e.g. moisture condition) of a material. The new method comprises next steps:
a. provide a relation, e.g. in the form of a mathematical or numerical model (or mechanism), between the electrical conductivity, measured at or from the surface of the material to be investigated, and its internal condition (e.g. moisture);
b. apply electrodes at the surface of the material to be examined and connect these with means by which the mutual electrical conductivity resp. resistance or impedance can be measured;
c. measure the mutual electrical conductivity resp. resistance or impedance for at least a part of the electrodes;
d. input the measured values into the said relation and output the obtained results.

Instead of using electrodes which physically penetrate into the material to be monitored, use is made of surface electrodes (which e.g. may be bonded to the surface without damaging the material e.g. with the help of electrical conductive glue), the measured values of which may be processed mathematically and/or numerically in a few steps into a two or three dimensional image of the moisture condition. The invention is based on the understanding that mathematical and numerical models for such a conversion of the electrical values obtained from the surface of the material into a two or three dimensional image, are sufficiently reliable to be useful for the present purpose. Besides, it will be indicated how such relations (models) may be applied, by which the measurement of electrical values at the surface of the object concerned can produce a reliable image of the internal material condition.

Preferably, the step mentioned under c. is repeated in the time, the successively measured values are input to said relation and the resulting outcomes, e.g. making use of a time dependent model, are related to each other. This way it is possible to use the measured values (data) to predict the course of the future internal material condition in the time. Optionally, both steps mentioned under c. and d. can be repeated (pair wise) in the time, and the results may be related to each other. So, in the first option a series of electrical conductivity values, measured at different moments, is- after being stored temporarily - supplied as a series of measured values to the relation and processed. In the second option the electrical conductivity values measured at different moments are supplied individually to the relation and processed.

The said internal condition of the material which influences the electrical conductivity, may refer to the (local) moisture of that material. However, the electrical conductivity to be monitored can also change under the influence of other physical, chemical or biological processes in the interior of the material, e.g. chemical degradation, rot (wood), rust (of e.g. concrete reinforcements), etc., which processes also can affect the internal condition of the material.

The measure electrodes preferably form a (geometrically) regular series, of which the electrical conductivity is measured, in pairs with increasing mutual electrode distance (e.g. between the electrodes 1-2, 1-3, 1-4 etc.). To avoid unwanted, e.g. electrochemical phenomena, preferably the mutual electrical conductivity is measured by means of an AC- source. To avoid disturbances by the public electricity network, the frequency of which is ca. 50 - 60 Hz, the frequency of the AC- source preferably is higher than the frequency of the electric mains, but not equal to a multiple of this frequency, because of failures by higher harmonics, e.g. between 100 and 150 Hertz.

Measurements like those proposed in the foregoing, fit to obtain an image of e.g. the internal moisture distribution within a material and its course during the time, preferably are combined with measurements which can be carried out with e.g. a sensor system with conventional sensors, fit for the measurement of the temperature, atmospheric humidity, condensation etc. All such measurements can be coupled to means for intelligent data processing by which the moisture distribution in porous building materials can be determined and predicted, if required correlated to the measured temperature, atmospheric humidity, condensation etc. In this way, using a combination of conventional sensors and the sensors discussed up here, all other relevant parameters which determine or influence the volume and changes in the moisture distribution within e.g. buildings, can be integrated in one monitor system, in which, with the help of a moisture transport model, proper predictions of the moisture distribution can be made.

It is noted that the electrodes at the surface of the material to be monitored (together forming one sensor) may form one series extending in a certain direction or two (or more) series, each extending in a certain direction. The sensor e.g. can generate two series of electrodes of which one extends in x-direction and the other in y-direction (e.g. perpendicular to the x-direction). If the sensor consists of electrodes only extending in x-direction, a two-dimensional (viz. in x-z-direction) moisture image can be obtained from that. If the sensor consists of a series of electrodes extending in x-direction and a series of electrodes extending in y-direction, a three-dimensional (viz. in x-y-z-direction) moisture image can be obtained.

### Exemplary embodiment

Figure 1 shows an exemplary embodiment of a configuration which is adapted for performing the method as presented in the foregoing paragraph.
Figures 2 and 3 show a current distribution and equipotential lines in an object having a homogeneous conductivity through the entire object; the configuration of figure 2 comprising electrodes at a small mutual distance, the configuration of figure 3 comprising electrodes at a larger mutual distance.
Figures 4a to 4e illustrate an optimizing procedure to be discussed below.
Figure 5 illustrates the course of the moisture condition of an object, in the time.

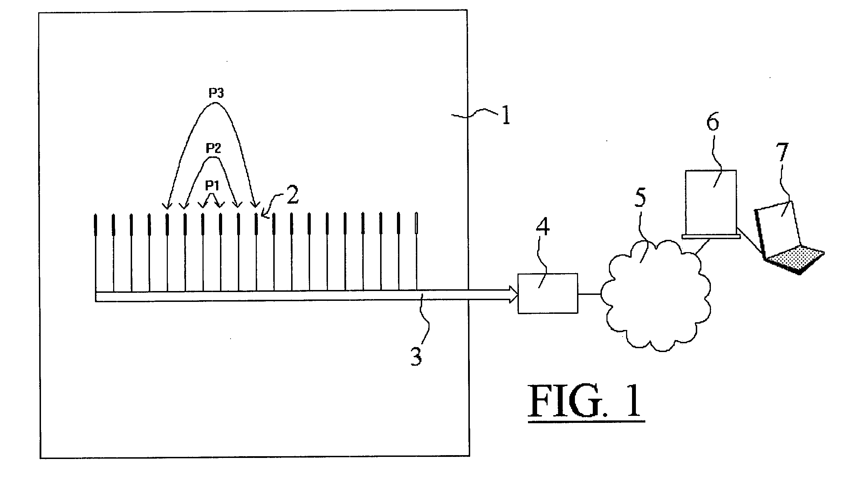

Figure 1 shows an object 1 of which the moisture has to be monitored resp. predicted by means of the method presented up here, for which end the means to be discussed below more in detail and additional methods can be used. The sensor comprises a number of electrodes, e.g. a number of strip shaped electrodes 2, or other forms, which are glued with an electricity conductive glue or the like, at certain distances at the surface of the material viz. the construction 1. The electrodes 2 can also extend in two directions (x- and y-direction, see foregoing), be circular or be placed in a concentric configuration. The length of the strip shaped electrodes 2, are preferably chosen small w.r.t. the surface of the building material 1 so there is talk of a local measurement. However, there can also be chosen for a more overall measurement with the help of relative large or long electrodes.

The mutual resistance or impedance values between the electrodes 2 are read out by a wire bundle or data bus 3 under control of a measuring module 4. For the sake of convenience, in this application text preferably will be spoken of impedance value, comprising both the reciprocal of the conductivity for DC and for AC, all the more since this value preferably is measured with the help of an AC- source - in module 4 - using a frequency of e.g. 140Hz (no interference with the electric mains), although with the present moisture measurement the impedance usually will have a substantially "ohmic" character. The mutual impedance values are then converted into a data signal by module 4, which data signal is transferred by a transmission network 5 to a processor 6. The processor reads in the conductivity data and processes these to data which can e.g. be printed or be displayed by a terminal 7. Anyway, it is noted that it will for sure be possible in the future to place the models at a chip or processor, by which the measuring data can also be interpreted locally. Below the operation of the processor 6 will be discussed more in detail.

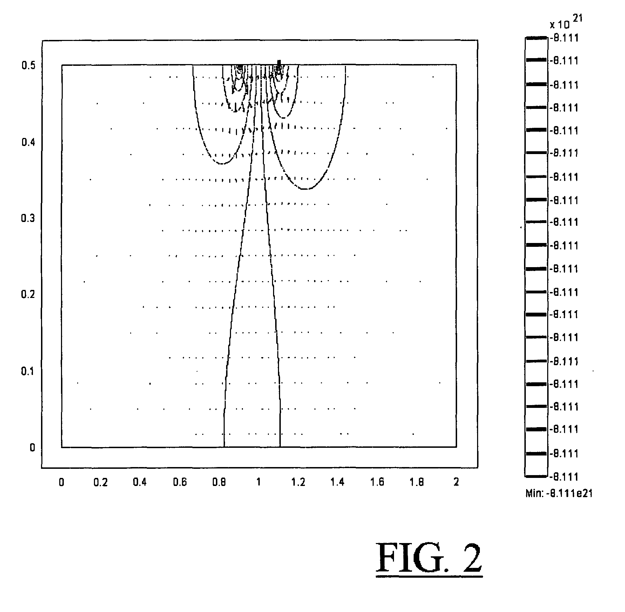

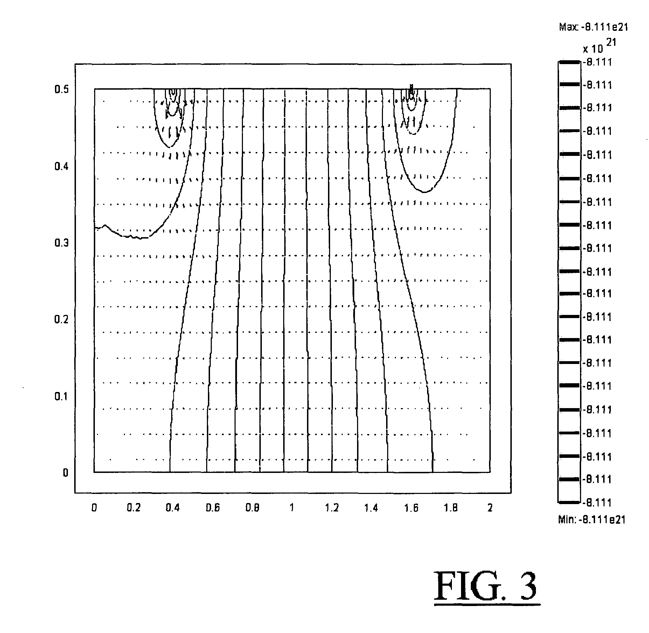

As an introduction to a discussion of the method which can be followed to convert the distribution of electrical conductivity at the surface of the object 1, in the direction in which the series of electrodes 2 extend (the x-direction), the figures 2 and 3 show the current density distribution and the equipotential lines in an arbitrary object having a constant, homogeneous conductivity (or impedance) through the entire object. Additionally, figure 2 shows a configuration comprising two electrodes at a small mutual distance, figure 3 comprising electrodes with a larger mutual distance.

The moisture distribution, which has to be determined by the system shown in figure 1, is considered, in contrast to the situation showed by figures 2 and 3, to be non-homogeneous in the z-direction i.e. the depth direction of the material. The measured impedance is determined by the geometrical configuration of the sensor (among other things the distance between the strip shaped electrodes 2) and the moisture distribution in the material 1. This last point can be clarified as follows.

An impedance or conductivity measurement can be done by measuring the current at a verified potential or electrical voltage (Ohm's law). The current distribution in the figures 2 and 3 shows that the electric current, for the electrodes at greater mutual distances, disperses deeper in the material than the electrodes which are more close to each other. This is because this, for both configurations, is the way of the least (total) impedance. In other words, the impedance of deeper situated layers has more effect to a current distribution for electrodes at greater distances and, in consequence, more effect to the impedance measured with these electrodes. It should be clear that a non-homogeneous impedance distribution in the z-direction has an other influence on the measured impedance for electrodes with a small and larger mutual distance respectively. Obtaining moisture profiles by the proposed sensor is based on this item. The electrical conductivity is preferably measured in pairs, with increasing mutual electrode distance, which is indicated in figure 1 by pairs of electrodes P1, P2, P3 etc.

Principally it is not possible to convert the measured impedance distribution straight-on via models or calibration curves into an impedance distribution in the z-direction. This is because for this conversion the conductivity distribution in the material is needed, and this is determined by the moisture distribution, and this moisture distribution just has to be determined. Hereinafter it will be explained how, by departing from a hypothetical (presupposed, presumed) moisture distribution, e.g. based on theoretical grounds, and as it were projecting back this presupposed moisture distribution to a matching theoretical conductivity- or impedance distribution, the real moisture distribution can be reduced. From this presumed distribution the matching measured values can be determined with the help of numerical methods. These calculated measured values can be confronted with the real (measured) values. By means of an adaptation and/or optimizing procedure ('fitting') the discrepancy between the real measured values and the calculated measured values (viz. from the presupposed moisture distribution) can be optimized (minimized), viz. by 'changing' the presupposed moisture distribution. This is an iterative process which finally gives a moisture distribution which results in a conductivity distribution which matches with the measured values. By means of such an optimization procedure a good determination of the moisture distribution in the z-direction can be obtained, based on the measured impedance distribution across the object surface.

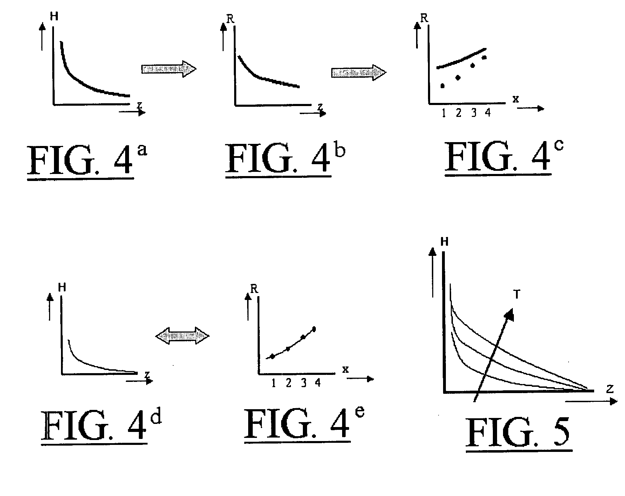

The optimizing procedure will be presented schematically in the figures 4a to 4e. First there is assumed a first theoretical 'guess' for the moisture distribution in the z-direction (figure 4a). Anyway, in the modern numerical optimizing software there exist methods for determining or adjusting such a good 'guess' . From this moisture distribution a 'theoretical' impedance distribution in the x-direction is determined, using conversion tables and/or algorithms (figure 4b) known from the literature. After that, departing from this electrical impedance distribution from figure 4b and the geometry of the sensor (figure 1), the measured values R of the sensor-electrodes 2 are calculated (figure 4c, continued line) with a numerical method, e.g. by means of a finite elements software package, . The difference between the calculated and the measured values (figure 4c, points) is minimized by means of an optimizing procedure by adjusting the moisture distribution (figure 4d and 4e.) In this way, the moisture profile is adjusted ('fitted') to the measured electrical impedance, until the 'best fit' has been obtained. (figure 4e). Numerical models for the conversion of the electrical impedance distribution in the z-direction to the x-direction, which can be linked to optimizing procedures, can e.g. be made in the known software package Matlab.

In foregoing way the momentaneous moisture distribution in the z-direction in the building material can be determined resulting from the measurements at the sensor However, by coupling the sensor 2 to an automated measuring system 4, possibly controlled by processor 6 via the network 5, measurements can be executed regularly (e.g. once an hour). By this, course of the moisture condition in the time can be followed, see figure 5. This course can be described by moisture transport models. Good calculating models can e.g. be placed in the form of a partial differential equation. As an input, such a model needs moisture transport parameters of the specific material and preconditions, such as the relative moisture of the open air, the temperature and condensation at the surface of the building material etc. Sensors for these quantities can be installed together with the sensor 2 (figure 1) on or in the neighbourhood of the object 1 and can be read out via the data bus 3 by module 4 and can, via network 5, be processed by processor 6 by which, by means of periodical measurements, the moisture transport parameters of the specific material of the object 1 can be determined.

With the so obtained data, and the moisture transport model (in the form of a numerical implementation of the partial differential equation), and assumptions about preconditions in the future, can be given a prediction of the moisture condition of the building material at any moment. With that a predicting moisture sensor has been obtained.

## Claims

1. Method for the examination of the internal condition of a material, comprising next steps:
a. provide a relation between the electrical conductivity, measured at the surface of the material to be examined (1), and its internal condition;
b. apply electrodes (2) at the surface of the material to be examined and connect means (3) to them which are fit to measure the mutual electrical conductivity;
c. measure for at least a part of the electrodes the mutual electrical conductivity;
d. input the measured values to said relation (6) and output the results from said relation.

2. Method according to claim 1, said relation comprising a mathematic model.

3. Method according to claim 1, said relation comprising a numerical model.

4. Method according to claim 1, the step mentioned under c. being repeated in the time and the successively measured values in said relation being input to said relation and the resulting outcomes being related to each other.

5. Method according to claim 1, the steps mentioned under c. and d. being repeated in the time and the outcomes which successively result from said relation being related to each other.

6. Method according to claim 1, the internal condition of the material referring to the moisture of that material.

7. Method 7 according to claim 1, the material being a more or less porous building material.

8. Method according to claim 1, the electrodes comprising a regular series and the conductivity being measured of electrode pairs of different mutual distance.

9. Method according to claim 1, the mutual electrical conductivity being measured with the help of an AC source.

10. Method according to claim 1, the frequency of the AC source being between 100 en 150 Hertz.

11. Method according to claim 1, comprising next steps:
- depart from a presumed distribution of the internal condition concerned;
- calculate, based on this presumed distribution of the internal condition, values for the electrical conductivity between the respective electrodes (2), which correspond with that presumed distribution;
- adjust the presumed distribution of the internal condition concerned as long as or in such way that the difference between the calculated values for the electrical conductivity between the respective electrodes and the measured values for the electrical conductivity between the respective electrodes is becoming minimal.

12. Method according to claim 1, said relation between the electrical conductivity measured at the surface of the material to be examined and its internal condition being completed with one or more relations between the internal condition of the material to be examined and other parameters.

13. Method according to claim 12, those other parameters referring to the temperature of the material to be examined and/or its surroundings and/or the moisture of the surroundings of the material to be examined and/or condensation values etc.

14. Method according to claim 12, the electrical conductivity at the surface of the material to be examined and said other parameters being correlated mutually and the correlations resulting from that being used in said relation between the electrical conductivity measured at the surface of the material to be examined and its internal condition.
